# EUROPÄISCHE PATENTSCHRIFT

(11) **EP 1 188 765 B1**
(45) Veröffentlichungstag und Bekanntmachung des Hinweises auf die Patenterteilung: **27.10.2004**
(21) Anmeldenummer: 01121386.5
(22) Anmeldetag: 02.08.1999
(51) Int. Cl.: C07K 5/062, C07K 5/065, C07K 5/083, A61K 38/05, A61K 38/06, A61P 3/00, A61P 35/00, A61P 37/00

(54) **Medizinische Verwendung von Mechanismus-orientierten Inhibitoren der Dipeptidylpeptidase I**
Medical Use of Mechanism-oriented inhibitors of dipeptidyl peptidase I
Utilisation medicale de Inhibiteurs orientés à méchanisme du peptidase dipeptidique I

(30) Priorität: 31.07.1998 DE 19834610
(43) Veröffentlichungstag der Anmeldung: 20.03.2002
(62) Teilanmeldung aus: 99115235.6
(73) Patentinhaber: Probiodrug AG, 06120 Halle/Saale (DE)
(72) Erfinder: Demuth, Hans-Ulrich,Dr., 06120 Halle/Saale (DE); Gerhartz, Bernd, Dr., 06120 Halle/Saale (DE); Heiser, Ulrich, Dr., 06120 Halle/Saale (DE); Schlenzig, Dagmar, 06120 Halle/Saale (DE)
(74) Vertreter: Forstmeyer, Dietmar, Dr. rer. nat., Dipl.-Chem.

(56) Entgegenhaltungen:
- DD-A- 158 109
- DEMUTH, HANS ULRICH ET AL: "Nitrogen-oxygen bond fission as the rate-determining step in the aqueous conversion of N-peptidyl-O-(p-nitrobenzoyl)hydroxylamine s to p-nitrobenzoic acid and peptidylhydroxamic acids" J. ORG. CHEM. (1989), 54(25), 5880-3, 1989, XP002131268
- DEMUTH, HANS ULRICH ET AL: "Inhibition of proteases with enkephalin-analog inhibitors" J. ENZYME INHIB. (1991), 4(4), 289-98, 1991, XP000876876
- SILBERRING, JERZY ET AL: "Inhibition of dynorphin converting enzymes from human spinal cord by N-peptidyl-O-acyl hydroxylamines" J. BIOCHEM. (TOKYO) (1993), 114(5), 648-51, 1993, XP002131269
- CHEROT, P. ET AL: "Enkephalin-degrading dipeptidylaminopeptidase: characterization of the active site and selective inhibition" MOL. PHARMACOL. (1986), 30(4), 338-44, 1986, XP000671433
- DEMUTH, HANS-ULRICH ET AL: "N-peptidyl, O-acyl hydroxamates: comparison of the selective inhibition of serine and cysteine proteinases" BIOCHIM. BIOPHYS. ACTA (1996), 1295(2), 179-186, 1996, XP000876878

## Beschreibung

Die vorliegende Erfindung betrifft die in den Ansprüchen definerte Verwendung von Verbindungen, die als spezifische Inhibitoren der Cysteinprotease Dipeptidyl Peptidase I (DP I) wirken. Diese Verbindungen werden durch die allgemeine Formel Formel A-B-NHO-Bz repräsentiert, in der A eine N-terminal ungeschützte Aminosäure außer Prolin und Hydroxyprolin ist, die in Peptidbindung mit B vorliegt, B eine Aminosäure außer Prolin, Hydroxyprolin oder Alanin ist und NHO eine Hydroxylamingruppierung darstellt, die in Amidbindung mit B sowie in Esterbindung mit Bz vorliegt, wobei Bz ein Benzoylrest ist, der unsubstituiert oder in ortho-, meta- und/oder para-Stellung substituiert ist.

Derartige Verbindungen zeichnen sich dadurch aus, daß sie in wäßrigen Lösungen, einschließlich biologischer Flüssigkeiten, chemisch stabil sind. Unmittelbar nach enzymatischer Einwirkung durch DP I auf diese Verbindungen entfalten diese ihre inhibitorische Aktivität gegenüber dem Targetenzym, was schließlich zur effizienten und vollständigen Inhibierung der DP I führt.

Von Dipeptidyl Peptidase I ist bekannt, daß sie aktive, granulozytäre Serinproteasen lymphatischer Zellen aus ihren Pro-Formen freisetzt, also bei Mechanismen mitwirkt, die physiologisch durch cytotoxische Lymphozyten bei der Immunabwehr genutzt werden. Bei pathophysiologischen Entartungen zellulärer Prozesse wie malignen Transformationen myeloider und lymphatischer Zellen kann die Suppression derartiger Mechanismen zur Behandlung von Krebs- oder Immunerkrankungen genutzt werden. Die erfindungsgemäßen Inhibitoren der DP I können zur Behandlung von derartigen pathophysiologischen Zuständen bzw. Erkrankungen eingesetzt werden.

Neben Proteasen, die in unspezifische Proteolyse einbezogen sind, was letztlich den Abbau von Proteinen zu Aminosäuren bewirkt, kennt man regulatorische Proteasen, die an der Funktionalisierung (Aktivierung, Deaktivierung, Modulierung) von endogenen Peptidwirkstoffen beteiligt sind (Kirschke *et al*., 1995) (Kräusslich & Wimmer, 1987). Insbesondere im Zusammenhang mit der Immunforschung und der Neuropeptidforschung sind eine Reihe solcher sogenannten Konvertasen, Signalpeptidasen oder Enkephalinasen entdeckt worden (Gomez *et al*., 1988)(Ansorge *et al*., 1991).

Dipeptidyl Peptidase I (DP I, Peptidase Klassifizierung Clan CA, Familie C1, IUBMB Enzym Klassifizierung EC 3.4.14.1, CAS Registration Nr. 9032-68-2) wurde von Gutman & Fruton 1948 entdeckt. DP I spaltet mit relativ breiter Substratspezifität sequentiell Dipeptide von unsubstituierten N-Termini von Polypeptidsubstraten ab (McDonald *et al.,* 1971; McDonald & Schwabe, 1977). DP I ist eine lysosomale Cysteinprotease, die durch die Abspaltung N-terminaler Dipeptide aktive Enzyme aus Proenzymen, wie Granzym A, Granzym B, Leukozyten Elastase, Cathepsin B, Neuraminidase in den lysosomalen Granula cytotoxischer T-Lymphozyten freisetzen kann (Kummer *et al*., 1996; Thiele & Lipsky, 1997.

Es wird daher vermutet, daß DP I in pathologische Mechanismen wie apoptotische Prozesse, Muskeldystrophie und Krebsentstehung involviert ist (Aoyagi *et al.*, 1983; Gelman *et al*., 1980; Schlangenauff *et al*., 1992; Shi *et al*., 1992).

DP I ist als Konvertase des Blutzucker-steigernden Hormons Glukagon bekannt, das bei enzymatisch verminderter Konzentration zu lebensbedrohlicher Hypoglykämie führen kann (McDonald, J.K *et al*., 1971).

DP I wird durch reversible und irreversible Cysteinproteaseinhibitoren wie Leupeptin bzw. E-64 nur schwach inhibiert (Nikawa *et al*., 1992). Stärkere reversible Inhibitoren sind Stefin A und Chicken Cystatin, Proteininhibitoren aus der Cystatin Superfamilie (Nicklin & Barrett, 1984). Eine spezifische Inhibierung wurde mit den *a priori* reaktiven *affinity label* vom Typ der Diazomethylketone und Sulphonylmethylketone erreicht (Angliker *et al*., 1989; Green & Shaw, 1981; Hanzlik, R.P. & Xing, R., 1998). In den letzten Jahren wurden weitere neuartige reversible DP I-Inhibitoren sowie irreversibel wirkende *affinity label* der DP I bekannt (Palmer *et. al*., 1998; Thiele *et al*., 1997).

Im Gegensatz zu derartigen reversiblen Inhibitoren, die aufgrund von Diffusionsprozessen nur kurzzeitige Wirkungen entfalten können, und den *in vitro* auf das Zielenzym zwar irreversibel wirkenden *affinity label*, die aber durch ihren *a priori* vorhandenen chemisch reaktiven Rest vor ihrer Interaktion mit dem Zielprotein mit anderen Nukleophilen oder Elektrophilen in biologischen Flüssigkeiten reagieren können, zeichnen sich mechanismus-orientierte Inhibitoren dadurch aus, daß sie nur durch das Zielenzym katalytisch angegriffen und erst dadurch aktiviert werden. Derartige Inhibitoren sind auch als Selbstmord-Inaktivatoren bekannt. Solche hocheffizienten Selbstmordinaktivatoren für Cysteinproteasen wurden mit der Klasse der N-Peptidyl, O-Acyl Hydroxlamine entwickelt (Brömme *et al*., 1996). Demuth *et al*. konnten zeigen, dass das N-Dipeptidyl-O-Benzoyl-Hydroxylamin Tyr-Gly-NHO-Bz das Enzym "DCE" (Dynorphin Converting Enzyme) nur schwach und reversibel inhibiert ( Demuth, H.U., Silbering, J. und Nyberg, F. (1991). Inhibition of proteases with enkephalin-analogue inhibitors. J. Enzyme Inhib. **4**(4), 289-298.). Inhibitoren der DP I wurden aus dieser Verbindungsklasse nicht abgeleitet, da sich DP I gegenüber typischen irreversiblen Cysteinproteaseinhibitoren wie z.B. E-64 inert verhält.

Weiterhin neigen N-terminal ungeschützte Dipeptid-Derivate zu rapider, intramolekularer Zersetzung.

Der Erfindung liegt daher der überraschende Befund zugrunde, daß sich Verbindungen der allgemeinen Formel A-B-NHO-Bz mit einer für die Dipeptidyl Peptidase I charakteristischen Substratstruktur als hochpotente und hydrolytisch stabile irreversible Selbstmordinaktivatoren der DP I erweisen. Im Vergleich zu bisher bekannten DP I-Inhibitoren sind diese Verbindungen außerordentlich potent.

Damit ist es möglich, selektive Inhibitoren der Dipeptidyl Peptidase I darzustellen, die sich durch hohe Stabilität und Selektivität gegenüber dem Zielenzym ausweisen. Derartige Verbindungen können zur Beeinflussung DP I-vermittelter pathologischer Prozesse dienen. Die erfindungsgemäßen Inhibitoren der DP I können in pharmazeutischen Formulierungen zur Behandlung von derartigen pathophysiologischen Zuständen bzw. Erkrankungen eingesetzt werden.

Verbindungen der Formel R₁-CO-NH-O-CO-R₂ sind an sich bekannt, vgl. DD 294 711, wobei die Reste R₁-CO- N-geschützte oder ungeschützte Aminosäurereste sowie N-geschützte oder ungeschützte Peptidylreste sein können; und die Reste R₂-CO- aliphatische, verzweigte, aromatische sowie aromatische substituierte Acylreste, wie substituierte Benzoylreste, unsubstituierte heterocyclische Acylreste, primäre oder sekundäre, aliphatische, verzweigt aliphatische, heterocyclische oder aromatische, substituierte oder unsubstituierte Amine oder C-terminal geschützte oder ungeschützte Aminosäuren oder Peptide sein können.

Es ist nun gefunden worden, daß Verbindungen der allgemeinen Formel A-B-NHO-Bz überraschend gut zur Inibierung von Dipeptidyl Peptidase I geeignet sind, wobei A eine N-terminal ungeschützte Aminosäure außer Prolin und Hydroxyprolin ist, die in Peptidbindung mit B vorliegt, B eine Aminosäure außer Prolin, Hydroxyprolin oder Alanin ist und NHO eine Hydroxylamingruppierung darstellt, die in Amidbindung mit B sowie in Esterbindung mit Bz vorliegt, wobei Bz ein Benzoylrest ist, der unsubstituiert oder in ortho-, meta- und/oder para-Stellung substituiert ist.

Es hat sich als besonders wichtig herausgestellt, daß die Aminosäure A N-terminal ungeschützt ist, da sonst keine Hemmung von DP I (Cathepsin C) erfolgt. Dieses ist auch deshalb überraschend, da N-terminal geschützte Verbindungen zwar keine Hemmung gegenüber DP I aber eine bessere Hemmung gegenüber Cathepsin B und L aufweisen als die ungeschützten Verbindungen, vgl. Tabelle 3.

Weiter ist gefunden worden, daß die Selektivität der Inaktivierung enorm gesteigert wird, wenn die NHO-Gruppe in Esterbindung mit einer Benzoylgruppe verknüpft ist, vgl. Tabelle 2.

Eine derartige Wirkung dieser Verbindung wird durch den bekannten Stand der Technik nicht nahegelegt.

Als besonders geeignet haben sich dabei Verbindungen herausgestellt, bei denen A ein Glycinrest ist; eine noch bessere Wirkung weisen Verbindungen auf, bei denen B zusätzlich eine Phenylalaninrest ist; eine besonders herausragende Wirkung weist also die Verbindung H-Gly-Phe-NHO-Bz auf.

Erfindungsgemäß können weitere pharmazeutische Zusammensetzungen her gestellt werden, die mindestens eine Verbindung, gegebenenfalls in Kombination mit an sich üblichen Trägern und/oder Adjurantien umfassen.

Die Verbindungen bzw. Zusammensetzungen können zur Inhibierung des Enzyms Dipeptidyl Peptidase I verwendet werden.

Insbesondere können sie zur Behandlung von Erkrankungen von Säugern verwendet werden, die durch Modulation der DP I-Aktivität in verschiedenen Zellen, Geweben und Organen beeinflußt werden können.

Sie sind zur Behandlung von DP I-vermittelten malignen Zell-Entartungen, Immun- und Stoffwechselerkrankungen des Menschen geeignet.

### Ausführungsbeispiele

### Beispiel 1:

### Synthese von N-(H-Gly-Phe-),O-Benzoyl Hydroxylamin (= H-Gly-Phe-NHO-Bz)

### BOC-Gly-Phe-OMe (3)

BOC-Gly-OH (1) und H-Phe-OMe*HCl (2) wurden nach üblichen peptidchemischen Methoden in der Kälte (-20 Grad Celsius) gekuppelt. (1) 3.48g (19.98mmol), (2) 4.32g (19.98mmol), Chlorameisensäureisobutylester, 2.6ml, N-Methylmorpholin 2.2ml, Tetrahydrofuran 30ml
Ausbeute: 6.04g (16.18mmol), 81.1%

Das Produkt wurde aus Essigester/Pentan kristallisiert und ohne weiter Reinigung weiter verwendet.

### BOC-Gly-Phe-NHOH (4)

Hydroxylamin Hydrochlorid 4.16g (60mmol) wurde in 90ml trockenem Methanol gelöst. 85,2ml frisch präparierte NaOMe Lösung (3.5M in absolutem Methanol) wurde tropfenweise zugegeben. Die Mischung wurde nach 20min Rühren abfiltriert und das Filtrat in eine gekühlte Lösung der Verbindung (3), 5.60g (15mmol) in 10ml Methanol, unter Rühren getropft. Nach 8h Rühren bei 0°C wurde das Lösungsmittel evaporiert und das verbleibende Öl in 10 ml Wasser aufgenommen und mit 2ml Ethylacetat extrahiert. Die wässrige Phase wurde mittels Zugabe von KHSO₄ auf pH 3 gebracht und erneut mit 15ml Ethylacetat extrahiert. Die organische Phase, die das Peptidylhydroxylamin (4) enthielt, wurde evaporiert und der Rückstand aus Methanol/Pentan umkristallisiert.
Ausbeute: 4.15g (13.35mmol), 89%, ESI-MS: 338 (M+H)

### BOC-Gly-Phe-NHO-Bz (5)

(4) 0.5g (1.5mmol) wurde in 10ml trockenem CHCl₃ gelöst und auf -5°C gekühlt. In diese Lösung wurde 0.18ml Trifluoressigsäure in 5ml CHCl₃ gegeben, bei nachfolgender tropfenweiser Zugabe über ca. 30 min von 285ml Benzoylchlorid, aufgelöst in 10ml CHCl₃. Die Mischung wurde danach unter Rühren in eiskalte HCl-Lösung (pH 3) gegeben, wobei sich ein Öl bildete. Das Öl wurde mittels Ethylacetat extrahiert. Nach Abdampfen des überschüssigen Ethylacetates wurde das Rohprodukt in Methanol/Petrolether umkristallisiert.
Ausbeute: 0.325g(0.73mmol), 48%

### H-Gly-Phe-NHO-Bz*HCl (6)

Die BOC-Schutzgruppe wurde mittels HCl/Eisessig abgespalten:. (5) 0.320g (0.725mmol), HCl 5ml (1N in Eisessig)
Ausbeute: 0.218g(0.63mmol), 87%, Fp: 118-120 °C, ESI-MS: 342.3 (M+H)
¹H-NMR (500MHz, DMSOD₆) δ(ppm) = 2.80 - 3.01, 3.20-3.31 (CH₂ δ Phe, dd); 4.71-4.82 (CH α Phe, t); 4.71-4.79 (CH α Gly, s); 7.01-7.15 (N⁺H₃ Gly d), 7.25-8.20 (CH- arom, m), 9.05-9.10 (NH Phe d), 12.30-12.95 (NHO- s)
¹³C-NMR (500MHz, DMSOD₆) δ(ppm)= 38.83 (CβPhe), 46.3 (Cα Gly), 52.36 (Cα Phe), 126.52 (C³Phe), 126.76 (C²Phe) 128.19 (C³benzoyl), 129.08 (C² benzoyl), 129.21 (C⁴ benzoyl), 129.43 (C⁴ Phe), 134.32 (C¹ benzoyl), 137.21 (C¹ Phe), 163.83 (C=O Gly), 163.87 (C=O benzoyl), 168.13 (C=O Phe)
gefunden: C: 56.19 H: 5.18 N: 10.23 C₁₈H₂₀O₄N₃ berechnet: C:57.29 H: 5.30 N: 11.14

### Beispiel 2:

Inaktivierung von Dipeptidyl Peptidase I aus verschiedenen Quellen mit H-Gly-Phe-NHO-Bz

**Tabelle 1:**

| | | |
|---|---|---|
| Zweiter-Ordnungs Geschwindigkeitskonstanten der Inaktivierung der DP I aus Rinder- bzw. humaner Niere mittels H-Gly-Phe-NHO-Bz | | |

| | **k**_{**inact**}**/K**_{**i**} **[s**^{**-1**}**M**^{**-1**}**]** | **std.err.** |
|---|---|---|
| **DP I**_{bovine} | 36729 | 2,3% |
| **DP I**ₕᵤₘ | 34979 | 2,9% |

### Beispiel 2:

Selektivität der Inaktivierung der DP I mittels verschiedener Inhibitoren der allgemeinen Formel A-B-NHO-C, bzw. verschiedener Cysteinproteasen mittels DP I-Inhibitoren

**Tabelle 2:**

| | | |
|---|---|---|
| Zweiter-Ordnungs-Geschwindigkeitskonstanten der Inaktivierung von DP I (k_{inact}/Kᵢ [s⁻¹M⁻¹]). In Klammern die jeweiligen Inhibitorkonzentrationen in M Nb - Nitrobenzoyl Bz - Benzoyl Bz-CH₃ - 4-Methyl-Benzoyl | | |

| | **DP I**_{**hum**} | **DP I**_{**bovine**} |
|---|---|---|
| H-Ile-Pro-NHO-Nb | 208 (2e⁻⁵) | 149 (2e⁻⁵) |
| H-Tyr-Gly-NHO-Bz | 3619 (1e⁻⁶) | 3948 (1e⁻⁶) |
| Z-Gly-Phe-NHO-Bz-CH₃ | keine Hemmung (2e⁻⁵) | keine Hemmung (2e⁻⁵) |
| Boc-Ala-Ala-Ala-NHO-Bz | keine Hemmung (1e⁻⁴) | keine Hemmung (1e⁻⁴) |
| Boc-Ala-Ala-NHO-Bz | keine Hemmung (1e⁻⁴) | keine Hemmung (1e⁻⁴) |
| Boc-Ala-Pro-Phe-NHO-Bz | keine Hemmung (1e⁻⁴) | keine Hemmung (1e⁻⁴) |

**Tabelle 3:**

| | | |
|---|---|---|
| Zweiter-Ordnungs-Geschwindigkeitskonstanten k_{inact}/Kᵢ [s⁻¹M⁻¹] der Inaktivierung von DP I, Cathepsin B und L durch N-terminal geschützte und ungeschützte Inhibitoren. | | |

| **Enzym** | **Z-Gly-Phe-NHO-Bz** | **H-Gly-Phe-NHO-Bz** |
|---|---|---|
| DP I (humane Niere) | Keine Hemmung | 34979 |
| Cathepsin B (hum. Leber) | 418 | 87 |
| Cathepsin L (hum. Leber) | 223 | 37 |

### Beispiel 3:

Schutz des Abbaus des Peptidhormons Glukagon durch Vorinkubation von DP I mit H-Gly-Phe-NHO-Bz

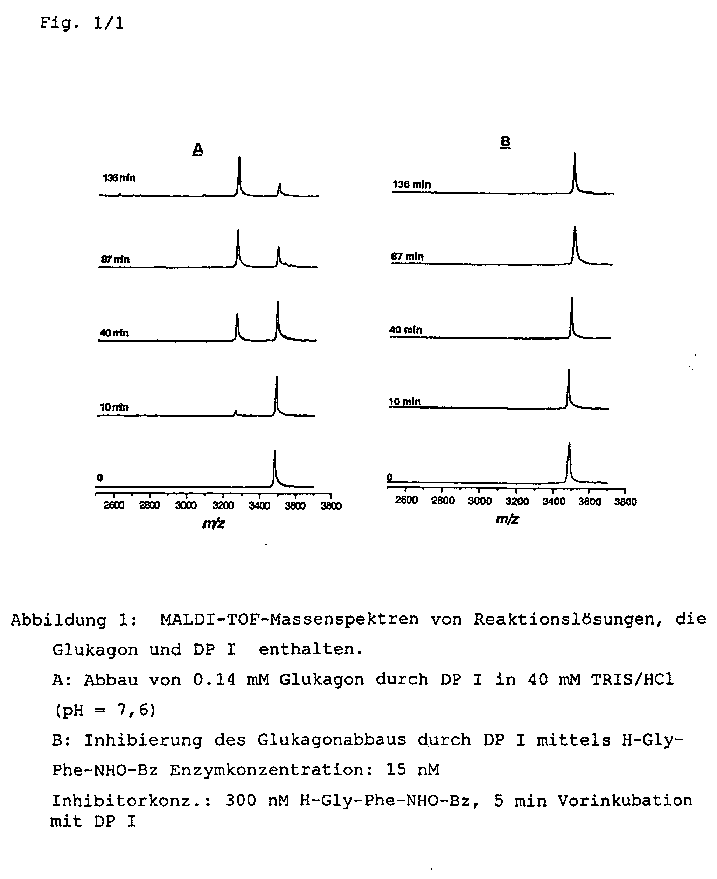

Abbildung 1: MALDI-TOF-Massenspektren von Reaktionslösungen, die Glukagon und DP I enthalten.
A: Abbau von 0.14 mM Glukagon durch DP I in 40 mM TRIS/HCl (pH = 7,6)
B: Inhibierung des Glukagonabbaus durch DP I mittels H-Gly-Phe-NHO-Bz Enzymkonzentration: 15 nM
Inhibitorkonz.: 300 nM H-Gly-Phe-NHO-Bz, 5 min Vorinkubation mit DP I

### Literaturverzeichnis

Ansorge, S., Schön, E., and Kunz, D. (1991). Membrane-bound peptidases of lymphocytes: functional implications. *Biomed.Biochim.Acta* **50,** 799-807.
Angliker, H., Wikstrom, P., Kirschke, H., and Shaw, E. (1989). The inactivation of the cysteinyl exopeptidases cathepsin H and C by affinity- labelling reagents. *Biochem. J.* **262**, 63- 68.
Aoyagi T., Wada, T., Kojima, F., Nagai, M., Miyoshino, S., and Umezawa, H. (1983). Two different modes of enzymatic changes in serum with progression of Duchenne muscular dystrophy. *Clin. Chim. Acta* **129**, 165-173.
Brömme, D., Neumann, U., Kirschke, H., and Demuth, H.-U. (1996). Novel N-peptidyl-O-acyl hydroxamates: selective inhibitors of cysteine proteinases. *Biochim. Biophys*. *Acta.* 1202, 271-276.
Gelman B.B., Papa, L., Davis, M.H., and Gruenstein, E. (1980). Decreased lysosomal dipeptidyl aminopeptidase I activity in cultured human skin fibroblasts in Duchenne's muscular dystrophy. *J. Clin. Invest.* **65**, 1398-1406.
Gomez, S., Gluschankof, P., Lepage, A., and Cohen, P. (1988). Relationship between endo- and exopeptidases in a processing enzyme system: activation of an endoprotease by the aminopeptidase B-like activity in somatostatin-28 convertase. *Proc Natl Acad Sci U S A* **85**, 5468-5472.
Green G.D.J. & Shaw, E. (1981). Peptidyl diazomethyl ketones are specific inactivators of thiol proteinases. *J. Biol. Chem.* **256,** 1923-1928.
Gutman H.R. & Fruton, J.S. (1948). On the proteolytic enzymes of animal tissues VIII. An intracellular enzyme related to chymotrypsin. *J. Biol. Chem.* **174**, 851-858.
Hanzlik, R.P. & Xing, R. (1998). Azapeptides as inhibitors and active site titrants for cysteine Proteinases. *J. Med. Chem.* **41,** 1344-1351.
Kirschke, H., Barrett, A.J., and Rawlings, N.D. (1995). Proteinases 1: lysosomal cysteine proteinases. *Protein Profile* **2,** 1581-1643.
Kräusslich, H.-G. and Wimmer, E. (1987). Viral Proteinases. *Ann. Rev. Biochem.* **57**, 701
Kummer, J.A., Kamp, A.M., Citarella, F., Horrevoets, A.J.G., and Hack, C.E. (1996). Expression of human recombinant granzyme A zymogen and its activation by the cysteine proteinase cathepsin C. *J. Biol. Chem.* **271,** 9281-9286.
McDonald, J.K, Callahan, P.X., Ellis, S., and Smith, R.E. (1971). Polypeptide degradation by dipeptidyl aminopeptidase I (cathepsin C) and related peptidases. In: Tissue Proteinases (Barrett, A.J. & Dingle, J.T., eds). Amsterdam: North-Holland Publishing, pp. 69-107.
McDonald, J.K. & Schwabe, C. (1977). Intracellular exopeptidases. In: Proteinases in Mammalian Cells and Tissues (Barrett, A.J., ed.). Amsterdam: North Holland Publishing, pp. 311-391.
Nicklin, M.J.H. & Barrett, A.J. (1984). Inhibition of cysteine proteinases and dipeptidyl peptidase I by eggwhite cystatin. *Biochem. J.* **223,** 245-253.
Nikawa, T., Towatari, T., and Katunuma, N. (1992). Purification and characterization of cathepsin J from rat liver. *Eur. J. Biochem.* **204,** 381-393.
Palmer, J. T., Rasnick, D., and Klaus, J.L. (1998). Reversible protease inhibitors. ***US-Pat.* 5,776,718**
Schlagenauff, B., Klessen, C., Teichmann-Dörr, S., Breuninger, H., and Rassner, G. (1992). Demonstration of proteases in basal cell carcinomas. A histochemical study using amino acid-4-methoxy-2-naphthylamides as chromogenic substrates. ***Cancer*** 70, 1133-1140.
Shi, L., Kam, C.-M., Powers, J.C., Aebersold, R., and Greenberg, A.H. (1992). Purification of three cytotoxic lymphocyte granule serine proteases that induce apoptosis through distinct substrate and target cell interactions. *J. Exp. Med.* **176**, 1521-1529.
Thiele, D.L., Lipsky, P.E., and McGuire, M.J. (1997). Dipeptidyl Peptidase-I inhibitors and uses thereof. ***US- Pat.* 5,602,102**

## Patentansprüche

1. Verwendung einer pharmazeutischen Zusammensetzung, die mindestens eine Verbindung der allgemeinen Formel A-B-NHO-Bz enthält, in der A eine N-terminal ungeschützte Aminosäure außer Prolin und Hydroxyprolin ist, die in Peptidbindung mit B vorliegt, B eine Aminosäure außer Prolin, Hydroxyprolin oder Alanin ist und NHO eine Hydroxylamingruppierung darstellt, die in Amidbindung mit B sowie in Esterbindung mit Bz vorliegt, wobei Bz ein Benzoylrest ist, der unsubstituiert oder in ortho-, meta- und/oder para-Stellung substituiert ist, gegebenenfalls in Kombination mit an sich üblichen Trägern und/oder Adjuvantien umfaßt, zur Herstellung eines Medikaments zur Behandlung von Dipeptidylpeptidase I (DPI)-vermittelten malignen Zell-Entartungen, Immun- und Stoffwechselerkrankungen des Menschen.

2. Verwendung einer pharmazeutischen Zusammensetzung nach Anspruch 1 zur Herstellung eines Medikaments zur Behandlung von apoptotischen Prozessen, Muskeldystrophie und Krebsentstehung.

3. Verwendung nach einem der vorstehenden Ansprüche, wobei der Benzoylrest mit F-, Cl-, Br-, NO₂-, NH₂-, CH₃-, oder CH₃O-Atomen bzw. Gruppen substituiert ist.

4. Verwendung nach einem der vorangegangenen Ansprüche, wobei A ein Glycinrest ist.

5. Verwendung nach einem der vorangegangenen Ansprüche, wobei B ein Phenylalaninrest ist.

6. Verwendung nach einem der vorangegangenen Ansprüche, wobei die verbindung H-Gly-Phe-NHO-Bz ist.

7. Verwendung nach einem der vorangegangenen Ansprüche, wobei der Benzoylrest unsubstituiert ist.

## Claims

1. Use of a pharmaceutical composition which comprises at least one compound corresponding to the general formula A-B-NHO-Bz, wherein A is an N-terminally unprotected amino acid except proline and hydroxyproline, which is peptide-bonded to B, B is an amino acid, except proline, hydroxyproline or alanine and NHO is a hydroxylamine grouping which is amide-bonded to B and ester-bonded to Bz, and Bz is a benzoyl radical which is unsubstituted or substituted in the ortho, meta or para position, optionally in combination with carriers and/or adjuvants that are customary *per se* for the production of a medicine for the treatment of malignant cell degeneration and immune and metabolic diseases in humans caused by dipeptidyl peptidase I (DP I).

2. Use of a pharmaceutical composition according to claim 1 for the production of a medicine for the treatment of apoptotic processes, muscular dystrophy and carcinogenesis.

3. Use according to any one of the preceding claims wherein the benzoyl radical is substituted with F, Cl, Br,NO₂, NH₂, CH₃ or CH₃O atoms or groups.

4. Use according to any one of the preceding claims wherein A is a glycine radical.

5. Use according to any one of the preceding claims wherein B is a phenylalanine radical.

6. Use according to any one of the preceding claims namely H-Gly-Phe-NHO-Bz.

7. Use according to any one of the preceding claims wherein the benzoyl radical is unsubstituted.

## Revendications

1. Utilisation d'une composition pharmaceutique comprenant au moins un composé de formule générale A-B-NHO-Bz, dans laquelle
A représente un résidu d'acide aminé N-terminal non-protégé à l'exception d'un résidu proline ou hydroxyproline, qui est lié par une liaison peptidique à B,
B représente un résidu d'acide aminé à l'exception d'un résidu proline, hydroxyproline ou alanine, et
NHO représente un groupe hydroxylamine, qui est lié à B par une liaison peptidique et à Bz par une liaison ester,
Bz représentant un résidu benzoyle non substitué ou portant un substituant en position ortho, méta ou para, éventuellement en combinaison avec des excipients et/ou adjuvants usuels pour la préparation d'un médicament destiné à traiter chez l'homme en particulier les transformations cellulaires malignes et les maladies du métabolisme et du système immunitaire liées à la dipeptidyle peptidase I.

2. Utilisation d'une composition pharmaceutique selon la revendication 1 pour la préparation d'un médicament destiné à traiter les processus apoptotiques, la dystrophie musculaire et le cancer.

3. Utilisation selon l'une quelconque des revendications précédentes dans laquelle le résidu benzoyle porte un substituant -F, -Cl, -Br, -NO₂, -NH₂, -CH₃ ou CH₃O-.

4. Utilisation selon l'une quelconque des revendications précédentes dans laquelle A représente un résidu glycine.

5. Utilisation selon l'une quelconque des revendications précédentes dans laquelle B représente un résidu phényle alanine.

6. Utilisation selon l'une quelconque des revendications précédentes dans laquelle le composé est H-Gly-Phe-NHO-Bz.

7. Utilisation selon l'une quelconque des revendications précédentes dans laquelle le résidu benzoyle est non substitué.
